# EUROPÄISCHE PATENTSCHRIFT

(11) **EP 2 498 726 B1**
(45) Veröffentlichungstag und Bekanntmachung des Hinweises auf die Patenterteilung: **07.05.2014**
(21) Anmeldenummer: 10782548.1
(22) Anmeldetag: 12.11.2010
(51) Int. Cl.: A61F 2/64, A61F 2/68

(54) **VERFAHREN UND VORRICHTUNG ZUR STEUERUNG EINES KÜNSTLICHEN ORTHETISCHEN ODER PROTHETISCHEN KNIEGELENKS**
METHOD AND DEVICE FOR CONTROLLING AN ARTIFICIAL ORTHOTIC OR PROSTHETIC KNEE JOINT
PROCÉDÉ ET DISPOSITIF POUR COMMANDER UNE ARTICULATION ORTHÉTIQUE OU PROTHÉTIQUE ARTIFICIELLE DU GENOU

(30) Priorität: 13.11.2009 DE 102009052895
(43) Veröffentlichungstag der Anmeldung: 19.09.2012
(73) Patentinhaber: Otto Bock Healthcare Products GmbH, 1070 Wien (AT)
(72) Erfinder: SEYR, Martin, A-1040 Wien (AT); PAWLIK, Roland, A-1130 Wien (AT); POP, Constantin, A-2630 Ternitz (AT); KAMPAS, Philipp, A-1020 Wien (AT)
(74) Vertreter: Stornebel, Kai
(86) Internationale Anmeldenummer: PCT/EP2010/006893
(87) Internationale Veröffentlichungsnummer: WO 2011/057792

(56) Entgegenhaltungen:
- EP-B1- 1 237 513
- DE-A1-102007 053 389

## Beschreibung

Die Erfindung betrifft ein Verfahren und eine Vorrichtung zur Steuerung eines künstlichen orthetischen oder prothetischen Gelenkes einer unteren Extremität mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Gelenkes über Sensoren Zustandsinformationen bereitgestellt werden. Das Verfahren und die Vorrichtung können neben der Verwendung in einem Prothesenkniegelenk unter anderem auch bei Hüftprothesen sinnvoll eingesetzt werden.

Bei einer Verwendung von Vorbringerfedern bei Hüftprothesen besteht das Problem, dass die Hüfte bei einer Entlastung beugt, was bei einem normalen Gangzyklus als Beugeerleichterung gewünscht ist, den Träger aber beim Rückwärtsgehen in eine ungewünschte Situation bringt.

Künstliche Gelenke, insbesondere Kniegelenke, für Orthesen oder Prothesen weisen ein oberes Anschlussteil und ein unteres Anschlussteil auf, die über eine Gelenkeinrichtung miteinander verbunden sind. An dem oberen Anschlussteil sind im Falle eines Kniegelenkes Aufnahmen für einen Oberschenkelstumpf oder eine Oberschenkelschiene angeordnet, während an dem unteren Anschlussteil ein Unterschenkelschaft oder eine Unterschenkelschiene angeordnet ist. Im einfachsten Fall ist das obere Anschlussteil mit dem unteren Anschlussteil durch ein einachsiges Gelenk verschwenkbar miteinander verbunden. Eine solche Anordnung ist nur in Ausnahmefällen ausreichend, um den gewünschten Erfolg, beispielsweise eine Unterstützung bei dem Einsatz einer Orthese oder ein natürliches Gangbild bei dem Einsatz in einer Prothese, zu gewährleisten.

Um die unterschiedlichen Anforderungen während der verschiedenen Phasen eines Schrittes oder bei anderen Verrichtungen möglichst natürlich darzustellen oder zu unterstützen, sind Widerstandseinrichtungen vorgesehen, die einen Flexionswiderstand und/oder einen Extensionswiderstand bereitstellen. Über den Flexionswiderstand wird eingestellt, wie leicht das untere Anschlussteil gegenüber dem oberen Anschlussteil in Flexionsrichtung verschwenkbar ist. Bei einem Kniegelenk wird daher über den Flexionswiderstand eingestellt, wie leicht der Unterschenkelschaft oder die Unterschenkelschiene im Verhältnis zu dem Oberschenkelschaft oder der Oberschenkelschiene bei einer aufgebrachten Kraft nach hinten schwingt. Der Extensionswiderstand bremst die Vorwärtsbewegung des Unterschenkelschaftes oder der Unterschenkelschiene und kann einen Streckanschlag ausbilden. Bei anderen Gelenktypen, wie dem Hüftgelenk oder dem Knöchelgelenk, gelten diese Ausführungen entsprechend den kinematischen Verhältnissen.

Über einstellbare Widerstandseinrichtungen ist es möglich, den jeweiligen Flexions- und/oder Extensionswiderstand an den Nutzer der prothetischen oder orthetischen Einrichtung anzupassen oder auf verschiedene Gang- oder Bewegungssituationen einzugehen, um bei sich verändernden Bedingungen einen angepassten Widerstand bereitstellen zu können.

Aus der DE 10 2008 008 284 A1 ist ein orthopädietechnisches Kniegelenk mit einem O-berteil und einen verschwenkbar daran angeordneten Unterteil bekannt, dem mehrere Sensoren zugeordnet sind, beispielsweise ein Beugewinkelsensor, ein Beschleunigungssensor, ein Neigungssensor und/oder ein Kraftsensor. Der Extensionsanschlag wird in Abhängigkeit von den ermittelten Sensordaten eingestellt.

Die DE 10 2006 021 802 A1 beschreibt eine Steuerung eines passiven Prothesenkniegelenkes mit verstellbarer Dämpfung in Flexionsrichtung zur Anpassung einer Protheseneinrichtung mit oberseitigen Anschlussmitteln und einem Verbindungselement zu einem Kunstfuß. Die Anpassung erfolgt an das Treppaufgehen, wobei ein momenten-armes Anheben des Prothesenfußes detektiert und die Flexionsdämpfung in einer Anhebephase auf unterhalb eines Niveaus, das für eine Gehen in der Ebene geeignet ist, abgesenkt wird. Die Flexionsdämpfung kann in Abhängigkeit von der Veränderung des Kniewinkels und in Abhängigkeit von der auf den Unterschenkel wirkenden Axialkraft angehoben werden.

Die DE 10 2007 053 389 A1 beschreibt ein Verfahren und eine Vorrichtung zur Steuerung eines orthopädischen Gelenkes einer unteren Extremität mit zumindest einem Freiheitsgrad mit einem verstellbaren Aktuator zur Anpassung einer orthopädischen Einrichtung, die oberseitige Anschlussmittel an eine Gliedmaße und ein distal zu Anschlussmitteln gelenkig angeordnetes orthopädisches Gelenk aufweist, an Gehsituationen, die von einem Gehen in der Ebene abweichen. Dabei werden mehrere Parameter der orthopädischen Einrichtung über Sensoren erfasst, die erfassten Parameter mit Kriterien, die anhand mehrerer Parameter und/oder Parameterverläufe erstellt worden und in einer Rechnereinheit abgelegt sind, verglichen und ein Kriterium ausgewählt, das anhand der ermittelten Parameter oder Parameterverläufe geeignet ist. Auf der Grundlage des gewählten Kriteriums werden Beugungswiderstände, Bewegungsumfänge, Antriebskräfte und/oder deren Verläufe eingestellt, um Sonderfunktionen zu steuern, die vom Gehen in der Ebene abweichen. Ein Kippwinkel eines Teils der orthopädischen Einrichtung im Raum und/oder ein Verlauf einer Kippwinkeländerung eines Teils der orthopädischen Einrichtung können als Parameter herangezogen werden.

Die EP 1237513 B1 beschreibt eine Prothese oder Orthese mit einer Steuerungseinrichtung und einem damit gekoppelten Sensor, der einen Neigungswinkel bezogen auf eine feststehende Linie eines mit einem Gelenk verbundenen Teils erfasst. Auf der Basis der Neigungswinkeldaten werden die Bewegungseigenschaften des Gelenks verändert, also das Gelenk abgebremst oder freigegeben.

Weiterhin sind aus dem Stand der Technik so genannte Bremskniegelenke bekannt, bei denen mechanisch bei wachsender Axialbelastung der Flexions- und Extensionswiderstand erhöht wird. Dies wird im einfachsten Fall dadurch erreicht, dass zwei Bremsflächen vorgesehen sind, die durch eine Bodenreaktionskraft aufeinander gepresst werden. Eine solche Ausgestaltung ist an der Bremseinrichtung ist für moderne Prothesenkniegelenke mit gesteuerten Widerstandseinrichtungen nicht einsetzbar.

Aufgabe der vorliegenden Erfindung ist es, ein Verfahren und eine Vorrichtung bereitzustellen, mit denen es möglich ist, das Knie automatisch beim Rückwärtsgehen mit einem erhöhten Widerstand zu belasten oder zu blockieren, ohne dass eine bewusste Aktivierung oder Deaktivierung des Modus erfolgen muss.

Erfindungsgemäß wird diese Aufgabe mit einem Verfahren gemäß dem Hauptanspruch und einer Vorrichtung gemäß dem nebengeordneten Anspruch erreicht. Vorteilhafte Ausgestaltungen und Weiterbildungen der Erfindung sind in den Unteransprüchen aufgeführt.

Das erfindungsgemäße Verfahren zur Steuerung eines künstlichen orthetischen oder prothetischen Kniegelenkes mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Gelenkes über Sensoren Zustandsinformationen bereit gestellt werden, sieht vor, dass der Beugewiderstand in der Standphase erhöht oder nicht verringert wird, wenn ein in Richtung auf die Vertikale abnehmender Inertialwinkel eines Unterschenkelteils und ein gleichzeitig belasteter Vorfuß ermittelt wird. Durch die Koppelung der Sensorgröße eines abnehmenden Inertialwinkels eines Unterschenkelteils in Richtung auf die Vertikale mit dem Vorhandensein einer Vorfußbelastung ist es möglich, das Rückwärtsgehen sicher zu detektieren und keine Schwungphase auszulösen, also den Flexionswiderstand nicht zu verringern, um eine ungewollte Beugung des Kniegelenkes zu vermeiden, wenn beim Rückwärtsgehen das versorgte Bein nach hinten gestellt und aufgesetzt wird. Dadurch ist es möglich, dass das versorgte Bein in Beugerichtung belastet wird, ohne einzuknicken, so dass es einem mit einer Prothese oder Orthese versorgten Patienten möglich ist, ohne eine gesonderte Verriegelung aktivieren zu müssen, rückwärts zu gehen. Sollte ein Rückwärtsgehen erkannt werden, ist es vorteilhaft, wenn der Beugewiderstand erhöht oder nicht verringert wird, so dass eine Schwungphasenauslösung auf jeden Fall unterbleibt.

Eine Weiterbildung der Erfindung sieht vor, dass der Widerstand erhöht oder zumindest nicht verringert wird, wenn die Inertialwinkelgeschwindigkeit eines Gelenkteils einen Schwellwert unterschreitet oder anders ausgedrückt, dass eine Schwungphase mit einer Absenkung des Flexionswiderstandes eingeleitet wird, wenn die Inertialwinkelgeschwindigkeit einen vorbestimmten Schwellenwert überschreitet. Ebenfalls ist es möglich, dass über die Bestimmung des Inertialwinkels eines Gelenkteils, insbesondere des Unterschenkelteils und der Inertialwinkelgeschwindigkeit eines Gelenkteils, insbesondere des Unterschenkelteils, ermittelt wird, dass sich der Prothesennutzer oder Orthesennutzer rückwärts bewegt und ein gegen eine Flexion verriegeltes oder hoch gebremstes Kniegelenk benötigt. Dementsprechend wird der Widerstand, sofern er noch nicht ausreichend hoch ist, erhöht, so dass das Kniegelenk gegebenenfalls blockiert werden kann.

Weiterhin kann vorgesehen sein, dass der Verlauf der Vorfußbelastung ermittelt wird und der Widerstand erhöht oder nicht verringert wird, wenn bei einem abnehmenden Inertialwinkel des Unterschenkelteils sich die Vorfußbelastung verringert. Während bei einer Vorwärtsbewegung nach dem Fersenstoß sich die Vorfußbelastung erst dann erhöht, wenn das Unterschenkelteil über die Vertikale hinaus nach vorne verschwenkt wurde, nimmt die Vorfußbelastung beim Rückwärtsgehen bei einem abnehmenden Inertialwinkel ab, so dass bei Vorliegen der beiden Zustände, nämlich einem abnehmenden Inertialwinkel und einer abnehmenden Vorfußbelastung auf ein Rückwärtsgehen geschlossen werden kann. Dementsprechend wird dann der Widerstand auf denjenigen Wert erhöht, der zum Rückwärtsgehen vorgesehen ist.

Es kann weiterhin vorgesehen sein, dass der Widerstand erhöht bzw. nicht verringert wird, wenn der Kniewinkel weniger als 15° beträgt. Dadurch wird ausgeschlossen, dass während der Schwungphase und bei einem gebeugten Knie bei entsprechenden Winkeln oder Winkelgeschwindigkeiten das Kniegelenk verriegelt und nicht mehr gebeugt werden kann. Das Rückwärtsgehen kann somit nur dann erfolgen, wenn sich das versorgte Bein in einer gestreckten oder nahezu gestreckten Stellung befindet. Es kann ebenfalls vorgesehen sein, dass der Widerstand erhöht oder nicht reduziert wird, obwohl der Kniewinkel mehr als 15° beträgt, wenn die Kniewinkelgeschwindigkeit sehr gering ist oder ein statischer Zustand vorhanden ist, also das versorgte Bein zurück gesetzt ist und keine Gehbewegung eingeleitet wird. In diesem statischen Fall ist es schwierig zu erkennen, ob eine Vorwärts- oder Rückwärtsbewegung stattfinden wird. '

Eine weitere Kenngröße kann das Kniemoment sein, das erfasst wird und als Grundlage dafür dient, ob der Widerstand erhöht oder nicht verringert wird. Wenn ein in Flexionsrichtung wirkendes Kniemoment ermittelt wird, also wenn der Prothesenfuß aufgesetzt wurde und ein Flexionsmoment im Knie detektiert wird, liegt eine Situation vor, bei der ein Rückwärtsgehen anzunehmen ist, so dass dann eine Flexionssperre, also ein Erhöhen des Widerstandes auf einen Wert, der ein Beugen ohne weiteres nicht ermöglicht, gerechtfertigt ist.

Der Inertialwinkel des Unterschenkelteils kann direkt über eine Sensoreinrichtung, die an dem Unterschenkelteil angeordnet ist, bestimmt werden oder aus dem Inertialwinkel eines anderen Anschlussteils, beispielsweise des Oberschenkelteils und einem gleichfalls ermittelten Gelenkwinkel. Da der Gelenkwinkel zwischen dem Oberschenkelteil und dem Unterschenkelteil auch für andere Steuerungssignale eingesetzt werden kann, ist durch die Mehrfachanordnung von Sensoren und die mehrfache Nutzung der Signale eine Redundanz gegeben, so dass auch bei Ausfall eines Sensors die Funktionalität der Prothese oder Orthese weiterhin erhalten bleibt. Eine Veränderung des Inertialwinkels eines Gelenkteiles kann direkt über ein Gyroskop oder aus der Differenzierung eines Inertialwinkelsignals des Gelenkteiles oder aus dem Inertialwinkelsignal eines Anschlussteiles und einem Gelenkwinkel ermittelt werden.

Der Flexionswiderstand kann in der Standphase auf einen für die Schwungphase geeigneten Wert reduziert werden, wenn ein relativ zur Vertikalen hin zunehmender Inertialwinkel des Unterschenkelteils ermittelt wird. Der zunehmende Inertialwinkel des Unterschenkelteils zeigt an, dass sich der Prothesennutzer oder Orthesennutzer in einer Vorwärtsbewegung befindet, wobei als Angelpunkt das distale Ende des Unterschenkelteils angenommen wird. Es ist vorgesehen, dass die Reduzierung nur dann erfolgt, wenn die Zunahme des Inertialwinkels über einem Schwellwert liegt. Weiterhin kann der Widerstand reduziert werden, wenn die Bewegung des Unterschenkelteils relativ zu dem Oberschenkelteil nicht beugend, also streckend ist oder konstant bleibt, was auf eine Vorwärtsbewegung deutet. Gleichfalls kann der Widerstand reduziert werden, wenn ein streckendes Kniemoment vorliegt.

Weiterhin kann vorgesehen sein, dass der Abstand des Bodenreaktionskraftvektors zu einem Gelenkteil ermittelt wird und der Widerstand dann reduziert wird, wenn ein Schwellwert des Abstandes überschritten wird, also wenn der Abstand des Bodenreaktionskraftvektors über einen Mindestabstand zu einem Gelenkteil liegt, beispielsweise zu der Längsachse des Unterschenkelteils in einer bestimmten Höhe oder zu der Schwenkachse des Kniegelenkes.

Der Widerstand kann wieder auf einen für die Schwungphase geeigneten Wert reduziert werden, wenn ermittelt wurde, dass sich das Kniemoment von streckend auf beugend verändert hat. Die Reduzierung erfolgt dabei unmittelbar nach der Veränderung des Kniemomentes von streckend auf beugend.

Weiterhin kann vorgesehen sein, dass der Widerstand nach einer Reduzierung wieder auf den Wert in der Standphase erhöht wird, wenn innerhalb einer festgelegten Zeit nach der Reduzierung des Widerstandes ein Schwellwert für einen Inertialwinkel einer Gelenkkomponente, für eine Inertialwinkelgeschwindigkeit, für eine Bodenreaktionskraft, für ein Gelenkmoment, für einen Gelenkwinkel oder für einen Abstand eines Kraftvektors zu einer Gelenkkomponente nicht erreicht wird. Anders ausgedrückt, das Gelenk wird wieder in den Standphasenzustand eingestellt, sollte nicht innerhalb einer festgelegten Zeit nach einem Wechsel in den Schwungphasenzustand tatsächlich eine Schwungphase festgestellt werden. Dies beruht darauf, dass die Schwungphasenauslösung bereits stattfindet, bevor die Fußspitze den Boden verlassen hat, um eine rechtzeitige Schwungphaseneinleitung zu ermöglichen. Sollte eine Schwungphase dann aber nicht eingeleitet werden, wie das zum Beispiel bei einer Zirkumduktionsbewegung der Fall ist, muss wieder auf den sichernden Standphasenwiderstand geschaltet werden. Hierzu ist ein Timer vorgesehen, der überprüft, ob innerhalb einer bestimmten Zeit ein erwarteter Wert für eine der oben bezeichneten Größen vorliegt. Der Widerstand bleibt reduziert, also die Schwungphase bleibt aktiviert, wenn eine Gelenkwinkelzunahme detektiert wird, also wenn tatsächlich eine Schwungphase eingeleitet wird. Ebenfalls ist es möglich, dass nach dem Erreichen des Schwellwertes und dem Freischalten der Schwungphase der Timer erst dann eingeschaltet wird, wenn ein zweiter Schwellwert, der kleiner als der erste Schwellwert ist, unterschritten wird. Es kann also vorgesehen sein, dass der Widerstand nach einer Reduzierung wieder auf den Wert für die Standphase erhöht wird, wenn nach der Reduzierung des Widerstandes und einem der Reduzierung folgenden Erreichen eines Schwellwertes für einen Inertialwinkel einer Gelenkkomponente, eine Inertialwinkelgeschwindigkeit, eine Bodenreaktionskraft, ein Gelenkmoment, einen Gelenkwinkel oder einen Abstand eines Kraftvektors zu einer Gelenkkomponente nicht innerhalb einer festgelegten Zeit ein weiterer Schwellwert für einen I-nertialwinkel, eine Inertialwinkelgeschwindigkeit, für eine Bodenreaktionskraft, für ein Gelenkmoment, für einen Gelenkwinkel oder für einen Abstand eines Kraftvektors zu einer Gelenkkomponente erreicht wird.

Um künstliche Gelenke auf der Grundlage von Sensordaten zu steuern, werden diejenigen Sensoren angeordnet, die gerade notwendig sind, um einen Sicherheitsstandard bei der Detektierung von Gangphasenübergängen zu gewährleisten. Werden über das Mindestmaß hinausgehende Sensoren verwendet, z.B. um den Sicherheitsstandard zu erhöhen, ermöglicht diese Redundanz von Sensoren, Steuerungen zu realisieren, die nicht alle in oder an dem Gelenk angeordneten Sensoren nutzen und dennoch einen Mindeststandard an Sicherheit einhalten. Es ist vorgesehen, dass die Redundanz der Sensoren genutzt wird, um alternative Steuerungen zu realisieren, die im Falle eines Ausfalles von Sensoren mit den noch arbeitenden Sensoren immer noch ein Gehen mit Schwungphase ermöglichen und einen Mindeststandard an Sicherheit bieten.

Eine Vorrichtung zur Durchführung des oben beschriebenen Verfahrens mit einer einstellbaren Widerstandseinrichtung, die zwischen zwei gelenkig aneinander gelagerten Komponenten eines künstlichen orthetischen oder prothetischen Kniegelenkes angeordnet ist, mit einer Steuereinrichtung und Sensoren, die Zustandsinformationen der Vorrichtung erfassen, sieht vor, dass eine Einstellvorrichtung vorgesehen ist und dass über die Einstellvorrichtung eine belastungsabhängige Widerstandsänderung aktivier- oder deaktivierbar ist. In Abhängigkeit von dem Vorhandensein oder der Abwesenheit einer Vorfußbelastung wird die Widerstandseinrichtung aktiviert oder deaktiviert. Dadurch kann neben einer automatischen Erkennung des Rückwärtsgehens und der automatischen Widerstandsanpassung auch eine bewusste Aktivierung des Rückwärtsgehmodus erreicht werden, ebenfalls ist es möglich, diesen Modus auszuschalten und aus dem Standardprogramm der Kniesteuerung herauszunehmen.

Nachfolgend wird ein Beispiel der Erfindung anhand der Figuren näher erläutert. Es zeigen:
- Figur 1 -: eine schematische Darstellung einer Prothese während des Rückwärtsgehens; sowie

- Figur 2 -: eine schematische Darstellung einer Prothese in gebeugter Stellung.

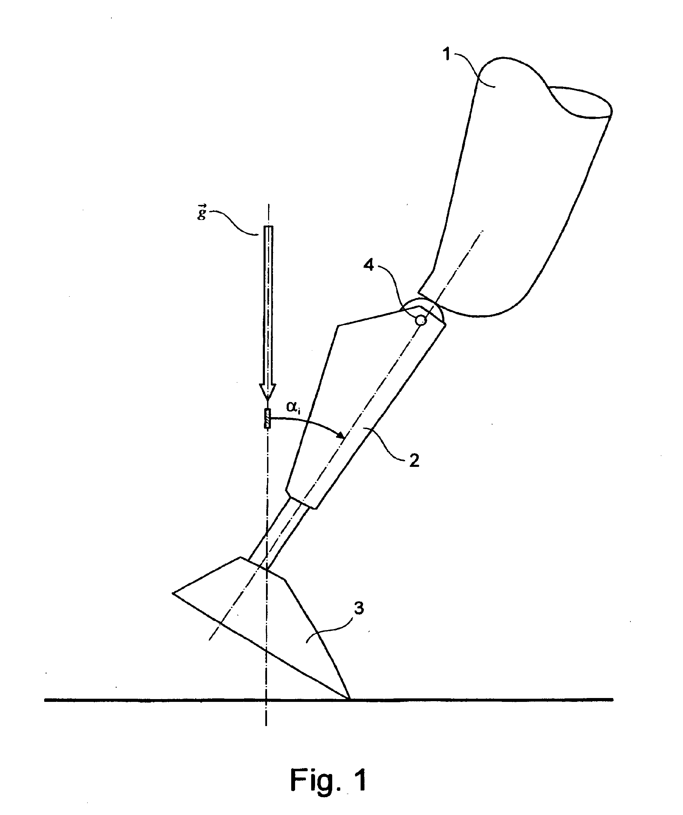

In der Figur 1 ist eine Prothese mit einem Oberschenkelteil 1 und einem gelenkig daran gelagerten Unterschenkelteil 2 gezeigt. An dem distalen Ende des Unterschenkelteils 2 ist ein Prothesenfuß 3 angeordnet. Das Unterschenkelteil 2 ist über ein Prothesenkniegelenk 4 gelenkig mit dem Oberschenkelteil 1 verbunden. Das Oberschenkelteil 1 ist in Gestalt eines Oberschenkelschaftes ausgebildet, so dass ein Oberschenkelstumpf eingeführt und darin gesichert werden kann. Der Inertialwinkel α_{I} ist der Absolutwinkel der Gelenkkomponente relativ zu der Vertikalen, woraus sich die Inertialwinkelgeschwindigkeit ω_{I} als Ableitung des Inertialwinkels nach der Zeit ergibt. Ausgehend von einer stehenden Situation wird beim Rückwärtsgehen das versorgte Bein, vorliegend die Prothese, nach hinten gesetzt, also der normalen Blickrichtung eines Prothesennutzers entgegengesetzt. Dadurch ergibt sich, dass der Inertialwinkel α_{I} des Unterschenkelteils 2 zu der Schwerkraftrichtung, die durch den Schwerkraftvektor g angedeutet ist, zunächst zunimmt, bis der Prothesenfuß 3 auf den Boden aufgesetzt wird. Nach dem Aufsetzen ist der Drehpunkt der Prothesenfuß. Wenn der Patient rückwärts geht, wird nach dem Aufsetzen der Inertialwinkel α_{I} abnehmen, die Inertialwinkelgeschwindigkeit ω_{I} somit negativ sein. Somit kann in dieser Situation über die Inertialwinkelgeschwindigkeit ω_{I} zwischen Vorwärtsgehen und Rückwärtsgehen unterschieden werden. Während beim Vorwärtsgehen in einer entsprechenden Situation, also bei Belastung des Vorfußes und nach vorne verkippter Prothese und positiver Inertialwinkelgeschwindigkeit ω_{I}, der Widerstand für die Schwungphase reduziert werden soll, soll der Widerstand beim Rückwärtsgehen in dieser Situation, also bei Belastung des Vorfußes und nach vorne verkippter Prothese und negativer Inertialwinkelgeschwindigkeit ω_{I}, nicht für eine Schwungphase reduziert werden. Der Inertialwinkel α_{I} ergibt sich z.B. wie eingezeichnet von der Vertikalen zur Längserstreckung des Unterschenkelteils 2. Als Drehpunkt oder Angelpunkt für die Bestimmung des zunehmenden Inertialwinkels α_{I} ist dabei das distale Ende des Unterschenkelteils 2 anzunehmen, so dass sich der Inertialwinkel α_{I} wie eingezeichnet von der Vertikalen zu der Längserstreckung des Unterschenkelteils 2 ergibt. Die Längserstreckung oder Längsachse des Unterschenkelteils 2 verläuft durch die Schwenkachse des Prothesenkniegelenkes 4 und bevorzugt ebenfalls durch eine Schwenkachse des Knöchelgelenkes oder aber zentral durch eine Anschlussstelle zwischen dem Prothesenfuß 3 und dem Unterschenkelteil 2. Der Inertialwinkel α_{I} des Unterschenkelteils 2 kann direkt durch eine an dem Unterschenkelteil 2 angeordnete Sensoreinrichtung bestimmt werden, alternativ dazu kann dies über einen Sensor an dem Oberschenkelteil 1 und einem Kniewinkelsensor bestimmt werden, der den Winkel zwischen dem Oberschenkelteil 1 und dem Unterschenkelteil 2 erfasst.

Zur Bestimmung der Inertialwinkelgeschwindigkeit wird die Veränderung des Inertialwinkels α_{I} über die Zeit ermittelt, so dass sich eine Winkelgeschwindigkeit ω_{I} ergibt, die mit Betrag und Richtung ermittelt werden kann. Liegt nun ein bestimmter Inertialwinkel α_{I} und eine bestimmte Inertialwinkelgeschwindigkeit ω_{I} vor, wird eine Schwungphase eingeleitet, falls ein bestimmter Schwellenwert für die Inertialwinkelgeschwindigkeit ω_{I} überschritten wird. Liegt ein abnehmender Inertialwinkel α_{I} vor und darüber hinaus noch eine Vorfußbelastung, kann auf ein Rückwärtsgehen geschlossen werden, so dass der Flexionswiderstand nicht verringert, sondern beibehalten oder erhöht wird, um keine Schwungphasenflexion einzuleiten.

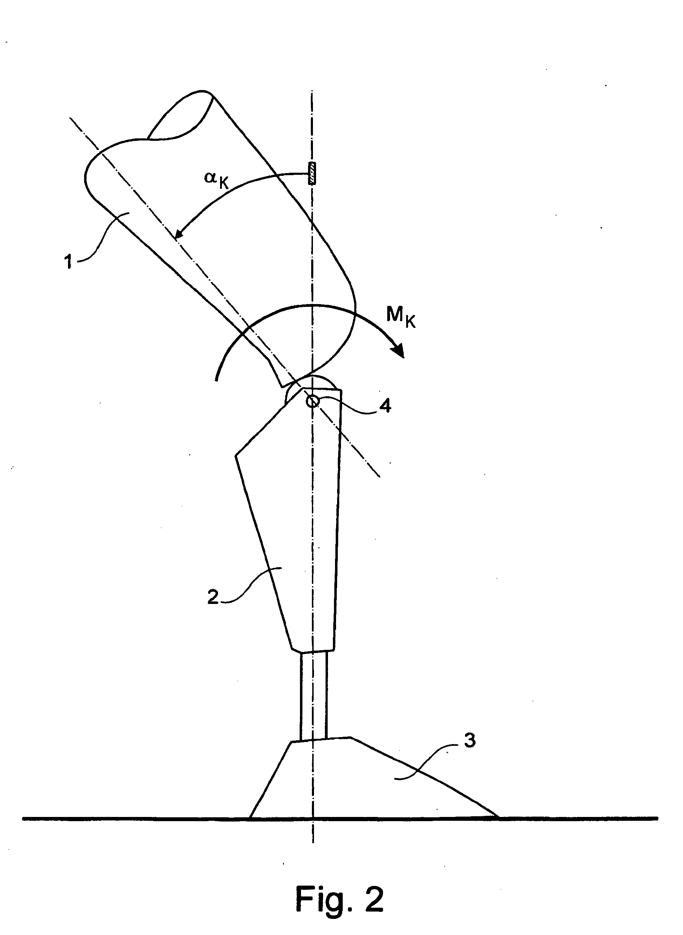

In der Figur 2 ist die Prothese in einem flach auf dem Boden aufgesetzten Zustand gezeigt und dient zur Vorzeichenkonvention in dieser Beschreibung. Bei anderen Vorzeichenkonventionen können sich entsprechend andere Bezeichnungen ergeben. Der Kniewinkel α_{K} entspricht dabei dem Winkel des Oberschenkelteils 1 zum Unterschenkelteil 2, entsprechend ergibt sich die Kniewinkelgeschwindigkeit ω_{K} aus der Ableitung des Kniewinkels α_{K} nach der Zeit. Um die Gelenkachse des Prothesenkniegelenkes 4 ist ein Kniemoment M_{K} wirksam, das in Streckrichtung als positiv wirkend angenommen wird. Liegt eine Gelenkwinkelgeschwindigkeit vor, wird davon ausgegangen, dass das Gelenk unter Belastung gebeugt wird. Damit würde eine Widerstandsverringerung eine schlagartig unsichere Situation verursachen und hat daher zu unterbleiben. Gleiches gilt bei einem zu großen Kniewinkel α_{K}, der anzeigt, dass die Prothese bereits gebeugt ist und wiederum eine Widerstandsverringerung eine schlagartig unsichere Situation verursachen würde und daher zu unterbleiben hat.

Darüber hinaus können noch andere Inertialwinkel definiert werden, die einen festgelegten Schwellwert erreichen müssen, damit eine Entscheidung über das Rückwärtsgehen oder das Einleiten einer Schwungphase getroffen wird. Hierbei können die Inertialwinkel des Unterschenkelteils 2, des Prothesenfußes 3 oder des Oberschenkelteils 1 herangezogen werden.

## Patentansprüche

1. Verfahren zur Steuerung eines künstlichen orthetischen oder prothetischen Gelenkes (4) einer unteren Extremität mit einer Widerstandseinrichtung, der zumindest ein Aktuator zugeordnet ist, über den der Beuge- und/oder Streckwiderstand in Abhängigkeit von Sensordaten verändert wird, wobei während der Benutzung des Gelenkes (4) über Sensoren Zustandsinformationen bereitgestellt werden, **dadurch gekennzeichnet, dass** der Beugewiderstand in der Standphase erhöht oder nicht verringert wird, wenn ein in Richtung auf die Vertikale abnehmender Inertialwinkel (α_{I}) eines Unterschenkelteils (2) und ein gleichzeitig belasteter Vorfuß ermittelt werden.

2. Verfahren nach Anspruch 1, **dadurch gekennzeichnet, dass** der Widerstand erhöht oder nicht verringert wird, wenn die Inertialwinkelgeschwindigkeit (ω_{I}) eines Gelenkteils (1, 2) einen Schwellwert unterschreitet.

3. Verfahren nach Anspruch 1 oder 2, **dadurch gekennzeichnet, dass** der Verlauf der Vorfußbelastung ermittelt wird und der Widerstand erhöht oder nicht verringert wird, wenn bei einem abnehmenden Inertialwinkel (α_{I}) des Unterschenkelteils (2) sich die Vorfußbelastung erhöht.

4. Verfahren nach einem der voranstehenden Ansprüche, **dadurch gekennzeichnet, dass** der Widerstand erhöht oder nicht verringert wird, wenn der Kniewinkel (α_{K}) weniger als 15° beträgt.

5. Verfahren nach einem der voranstehenden Ansprüche, **dadurch gekennzeichnet, dass** ein Kniemoment (M_{K}) erfasst und der Widerstand erhöht oder nicht verringert wird, wenn ein in Flexionsrichtung wirkendes Kniemoment (M_{K}) ermittelt wird.

6. Verfahren nach einem der voranstehenden Ansprüche, **dadurch gekennzeichnet, dass** der Inertialwinkel (α_{I}) des Unterschenkelteils (2) entweder unmittelbar oder aus dem Inertialwinkel (α_{I}) eines anderen Anschlussteiles (1, 3) und einem Gelenkwinkel ermittelt wird.

7. Verfahren nach einem der voranstehenden Ansprüche, **dadurch gekennzeichnet, dass** eine Veränderung des Inertialwinkels (αᵢ) eines Gelenkteiles (1, 2) direkt über ein Gyroskop oder aus der Differenzierung eines Inertialwinkelsignals des Gelenkteiles (1, 2, 3) oder aus dem Inertialwinkelsignal eines Anschlussteiles und einem Gelenkwinkel (α_{K}) ermittelt wird.

8. Verfahren nach einem der voranstehenden Ansprüche, **dadurch gekennzeichnet, dass** der Widerstand in der Standphase reduziert wird, wenn ein relativ zur Vertikalen zunehmender Inertialwinkel (α_{I}) des Unterschenkelteils (2) ermittelt wird.

9. Verfahren nach einem der voranstehenden Ansprüche, **dadurch gekennzeichnet, dass** der Widerstand reduziert wird, wenn die Bewegung des Unterschenkelteils (2) relativ zu dem Oberschenkelteil (1) nicht beugend ist.

10. Verfahren nach einem der voranstehenden Ansprüche, **dadurch gekennzeichnet, dass** der Widerstand reduziert wird, wenn ein streckendes Kniemoment (M_{K}) vorliegt.

11. Verfahren nach einem der voranstehenden Ansprüche, **dadurch gekennzeichnet, dass** der Abstand des Bodenreaktionskraftvektors zu einem Gelenkteil (1, 2, 3) ermittelt wird und der Widerstand reduziert wird, wenn ein Schwellwert des Abstandes überschritten wird.

12. Verfahren nach einem der voranstehenden Ansprüche, **dadurch gekennzeichnet, dass** der Widerstand nach einer Reduzierung wieder auf den Wert für die Standphase erhöht wird, wenn innerhalb einer festgelegten Zeit nach der Reduzierung des Widerstandes ein Schwellwert für einen Inertialwinkel (αᵢ) einer Gelenkkomponente (1, 2, 3), für eine Inertialwinkelgeschwindigkeit (ωᵢ), für eine Bodenreaktionskraft, für ein Gelenkmoment (M_{K}), für einen Gelenkwinkel (α_{K}) oder für einen Abstand eines Kraftvektors zu einer Gelenkkomponente (1, 2, 3) nicht erreicht wird.

13. Verfahren nach einem der voranstehenden Ansprüche, **dadurch gekennzeichnet, dass** der Widerstand nach einer Reduzierung wieder auf den Wert für die Standphase erhöht wird, wenn nach der Reduzierung des Widerstandes und einem der Reduzierung folgenden Erreichen eines Schwellwertes für einen Inertialwinkel (α_{I}) einer Gelenkkomponente, eine Inertialwinkelgeschwindigkeit (ωᵢ), eine Bodenreaktionskraft, ein Gelenkmoment (M_{K}), einen Gelenkwinkel (α_{K}) oder einen Abstand eines Kraftvektors zu einer Gelenkkomponente (1, 2, 3) nicht innerhalb einer festgelegten Zeit ein weiterer Schwellwert für einen Inertialwinkel (α_{I}), eine Inertialwinkelgeschwindigkeit (ωᵢ), für eine Bodenreaktionskraft, für ein Gelenkmoment (M_{K}), für einen Gelenkwinkel (α_{K}) oder für einen Abstand eines Kraftvektors zu einer Gelenkkomponente (1, 2, 3) erreicht wird.

14. Verfahren nach Anspruch 12 oder 13, **dadurch gekennzeichnet, dass** der Widerstand reduziert bleibt, wenn eine Gelenkwinkelzunahme detektiert wird.

15. Verfahren nach einem der voranstehenden Ansprüche, **dadurch gekennzeichnet, dass** im Falle eines Ausfalles von Einrichtungen zur Erfassung von Momenten (M_{K}), Kräften und/oder Gelenkwinkeln (α_{K}) alternative Steueralgorithmen auf Basis der restlichen Einrichtungen zur Veränderung des Streck- und/oder Beugewiderstandes verwendet werden.

16. Vorrichtung zur Durchführung des Verfahrens nach einem der voranstehenden Ansprüche, mit einer einstellbaren Widerstandseinrichtung, der zumindest eine Einstelleinrichtung zugeordnet ist, wobei die Widerstandseinrichtung zwischen zwei gelenkig aneinander gelagerten Komponenten (1, 2) eines künstlichen orthetischen oder prothetischen Kniegelenkes (4) angeordnet ist, mit einer Steuereinrichtung und Sensoren, die Zustandsinformationen der Vorrichtung erfassen, **dadurch gekennzeichnet, dass** eine Einstellvorrichtung vorgesehen ist und dass über die Einstellvorrichtung eine belastungsabhängige Widerstandsänderung aktivierbar und/oder deaktivierbar ist.

## Claims

1. A method for controlling an artificial orthotic or prosthetic joint (4) of a lower extremity with a resistance device, which is assigned at least one actuator by way of which the bending and/or stretching resistance is changed in dependence on sensor data, information pertaining to the state being provided by way of sensors during the use of the joint (4), **characterized in that** the bending resistance is increased, or not reduced, in the standing phase if an inertial angle (α_{I}) of a lower leg part (2) that is decreasing in the direction of the vertical and a simultaneously loaded forefoot are determined.

2. The method as claimed in claim 1, **characterized in that** the resistance is increased, or not reduced, if the inertial angle velocity (ωᵢ) of a joint part falls below a threshold value.

3. The method as claimed in claim 1 or 2, **characterized in that** the variation in the loading of the forefoot is determined and the resistance is increased, or not reduced, if, with a decreasing inertial angle (α_{I}) of the lower leg part (2), the loading of the forefoot is increased.

4. The method as claimed in one of the preceding claims, **characterized in that** the resistance is increased, or not reduced, if the knee angle (α_{K}) is less than 15°.

5. The method as claimed in one of the preceding claims, **characterized in that** a knee torque (M_{K}) is detected and the resistance is increased, or not reduced, if a knee torque (M_{K}) acting in the direction of flexion is determined.

6. The method as claimed in one of the preceding claims, **characterized in that** the inertial angle (α_{I}) of the lower leg part (2) is determined either directly or from the inertial angle (α_{I}) of another connection part (1, 3) and a joint angle.

7. The method as claimed in one of the preceding claims, **characterized in that** a changing of the inertial angle (α_{I}) of a joint part (1, 2) is determined directly by way of a gyroscope or from the differentiation of an inertial angle signal of the joint part (1, 2, 3) or from the inertial angle signal of a connection part and a joint angle (α_{K}).

8. The method as claimed in one of the preceding claims, **characterized in that** the resistance is reduced in the standing phase if an inertial angle (α_{I}) of the lower leg part (2) that is increasing in relation to the vertical is determined.

9. The method as claimed in one of the preceding claims, **characterized in that** the resistance is reduced if the movement of the lower leg part (2) in relation to the upper leg part (1) is not bending.

10. The method as claimed in one of the preceding claims, **characterized in that** the resistance is reduced if there is a stretching knee torque (M_{K}).

11. The method as claimed in one of the preceding claims, **characterized in that** the distance of the ground reaction force vector from a joint part (1, 2, 3) is determined and the resistance is reduced if a threshold value of the distance is exceeded.

12. The method as claimed in one of the preceding claims, **characterized in that**, after a reduction, the resistance is increased again to the value for the standing phase if, within a fixed time after the reduction of the resistance, a threshold value for an inertial angle (α_{I}) of a joint component (1, 2, 3), for an inertial angle velocity (ωᵢ), for a ground reaction force, for a joint torque (M_{K}), for a joint angle (α_{K}) or for a distance of a force vector from a joint component (1, 2, 3) is not reached.

13. The method as claimed in one of the preceding claims, **characterized in that**, after a reduction, the resistance is increased again to the value for the standing phase if, after the reduction of the resistance and reaching a threshold value for an inertial angle (α_{I}) of a joint component, an inertial angle velocity (ωᵢ), a ground reaction force, a joint torque (M_{K}), a joint angle (α_{K}) or a distance of a force vector from a joint component (1, 2, 3) after the reduction, a further threshold value for an inertial angle (α_{I}), for an inertial angle velocity (ωᵢ), for a ground reaction force, for a joint torque (M_{K}), for a joint angle (α_{K}) or for a distance of a force vector from a joint component (1, 2, 3) is not reached within a fixed time.

14. The method as claimed in claim 12 or 13, **characterized in that** the resistance remains reduced if a joint angle increase is detected.

15. The method as claimed in one of the preceding claims, **characterized in that**, in the case of a failure of devices for detecting torques (M_{K}), forces and/or joint angles (α_{K}), alternative control algorithms on the basis of the remaining devices are used for changing the stretching and/or bending resistance.

16. A device for carrying out the method as claimed in one of the preceding claims, with a settable resistance device which is assigned at least one setting device, whereas the resistance device is arranged between two components (1, 2) of an artificial orthotic or prosthetic knee joint that are mounted one against the other in a jointed manner, with a control device and sensors that detect information pertaining to the state of the device, **characterized in that** a setting device is provided and **in that** a loading-dependent change in resistance can be activated and/or can be deactivated by way of the setting device.

## Revendications

1. Procédé pour commander une articulation orthétique ou prothétique artificielle (4) d'une extrémité inférieure avec un système à résistance auquel est associé au moins un actionneur au moyen duquel on modifie la résistance à la flexion et/ou à l'extension en fonction de données sensorielles, et pendant l'utilisation de l'articulation (4) on prépare des informations d'état via des capteurs, **caractérisé en ce que** la résistance à la flexion est augmentée ou n'est pas réduite dans la phase de station debout lorsque l'on détermine qu'un angle d'inertie (σ₁) d'une partie formant jambe (2) diminue en direction de la verticale, et simultanément que la partie avant du pied est chargée.

2. Procédé selon la revendication 1, **caractérisé en ce que** la résistance est augmentée ou n'est pas diminuée quand la vitesse angulaire d'inertie (ω₁) d'une partie d'articulation (1, 2) passe au-dessous d'une valeur seuil.

3. Procédé selon la revendication 1 ou 2, **caractérisé en ce que** l'on détermine l'évolution de la charge de la partie avant du pied et la résistance est augmentée ou n'est pas réduite lorsque la charge sur l'avant du pied augmente tandis que l'angle d'inertie (σ₁) de la partie formant jambe (2) diminue.

4. Procédé selon l'une des revendications précédentes, **caractérisé en ce que** la résistance est augmentée ou n'est pas diminuée quand l'angle au genou (σ_{K}) est inférieur à 15°.

5. Procédé selon l'une des revendications précédentes, **caractérisé en ce que** l'on détecte un moment au genou (M_{K}), et la résistance est augmentée ou n'est pas réduite quand on détermine un couple au genou (M_{K}) agissant dans la direction de flexion.

6. Procédé selon l'une des revendications précédentes, **caractérisé en ce que** l'angle d'inertie (σ₁) de la partie formant jambe (2) est déterminé soit directement soit à partir de l'angle d'inertie (σ₁) d'une autre pièce de raccordement (1, 3) et d'un angle d'articulation.

7. Procédé selon l'une des revendications précédentes, **caractérisé en ce qu'**une modification de l'angle d'inertie (σ₁) d'une partie d'articulation (1, 2) est déterminée soit directement via un gyroscope, soit à partir de la différenciation d'un signal d'angle d'inertie de la partie d'articulation (1, 2, 3), soit encore à partir du signal d'angle d'inertie d'une pièce de raccordement et d'un angle d'articulation (σ_{K}).

8. Procédé selon l'une des revendications précédentes, **caractérisé en ce que** la résistance est réduite dans la phase en station debout quand on détermine qu'un angle d'inertie (s1) de la partie formant jambe (2) augmente par rapport à la verticale.

9. Procédé selon l'une des revendications précédentes, **caractérisé en ce que** la résistance est réduite quand le mouvement de la partie formant jambe (2) par rapport à la partie formant cuisse (1) n'est pas une flexion.

10. Procédé selon l'une des revendications précédentes, **caractérisé en ce que** la résistance est réduite lorsqu'on est en présence d'un moment au genou (M_{K}) en extension.

11. Procédé selon l'une des revendications précédentes, **caractérisé en ce que** la distance du vecteur de force de réaction au sol par rapport à une partie d'articulation (1, 2, 3) est déterminée et la résistance est réduite quand la distance passe au-dessus d'une valeur seuil.

12. Procédé selon l'une des revendications précédentes, **caractérisé en ce que**, après une réduction, la résistance est à nouveau augmentée à la valeur pour la phase en station debout si à l'intérieur d'un temps fixé après réduction de la résistance on n'atteint pas une valeur seuil pour un angle d'inertie (σ₁) d'une composante (1, 2, 3) de l'articulation pour une vitesse angulaire inertielle (ω₁), pour une force de réaction au sol, pour un moment à l'articulation (M_{K}), pour un angle à l'articulation (σ_{K}), ou pour une distance entre un vecteur de force et une composante (1, 2, 3) de l'articulation.

13. Procédé selon l'une des revendications précédentes, **caractérisé en ce que**, après une réduction, la résistance est à nouveau augmentée à la valeur pour la phase en station debout si après la réduction de la résistance et après avoir atteint suite à la réduction une valeur seuil pour un angle d'inertie (σ₁) d'une composante de l'articulation, une vitesse angulaire d'inertie (ω₁), une force de réaction au sol, un moment d'articulation (M_{K}), un angle d'articulation (σ_{K}) ou une distance d'un vecteur de force par rapport à une composante (1, 2, 3) de l'articulation, on n'atteint pas à l'intérieur d'un temps fixé une autre valeur seuil pour un angle d'inertie (σ₁), une vitesse angulaire d'inertie (ω₁), pour une force de réaction au sol, pour un moment d'articulation (M_{K}), pour un angle d'articulation (σ_{K}) ou pour une distance d'un vecteur de force par rapport à une composante (1, 2, 3) de l'articulation.

14. Procédé selon la revendication 12 ou 13, **caractérisé en ce que** la résistance reste réduite quand on détecte une augmentation de l'angle d'articulation.

15. Procédé selon l'une des revendications précédentes, **caractérisé en ce que**, dans le cas d'une panne de systèmes pour détecter des moments (MK), des forces et/ou des angles d'articulation (σ_{K}), on utilise en variante des algorithmes de commande sur la base des systèmes restants pour faire varier la résistance à l'extension et/ou à flexion.

16. Appareil pour mettre en oeuvre le procédé selon l'une des revendications précédentes, comprenant un système à résistance réglable, auquel est associé au moins un dispositif de réglage, dans lequel le système à résistance est agencé entre deux composantes (1, 2) montées l'une avec l'autre de façon articulée, d'une articulation orthétique ou prothétique artificielle du genou (4), avec un système de commande et des capteurs qui détectent des informations d'état de l'appareil,
**caractérisé en ce qu'**il est possible d'activer et/ou de désactiver une modification de résistance en fonction de la charge via le dispositif de réglage.
